(19) Europäisches Patentamt
European Patent Office
Office européen des brevets

(11) EP 4 729 549 A1

(12) EUROPEAN PATENT APPLICATION

(43) Date of publication:
**22.04.2026 Bulletin 2026/17**

(21) Application number: **24306756.8**

(22) Date of filing: **21.10.2024**

(51) International Patent Classification (IPC):
***C08B 31/04*** *(2006.01)* ***C08B 37/00*** *(2006.01)*
***C08L 3/02*** *(2006.01)* ***C08L 3/06*** *(2006.01)*
***C08L 5/00*** *(2006.01)* ***A61K 8/73*** *(2006.01)*
***D21H 17/24*** *(2006.01)* ***D21H 17/28*** *(2006.01)*
***D21H 17/31*** *(2006.01)* ***D21H 21/10*** *(2006.01)*
***D21H 23/04*** *(2006.01)* ***C02F 1/56*** *(2023.01)*

(52) Cooperative Patent Classification (CPC):
**C08B 31/04; A61K 8/73; A61K 8/732; A61Q 5/02; A61Q 5/12; C08B 31/12; C08B 37/0033; C08L 3/02; C08L 3/06; C08L 3/08; C08L 5/00; D21H 17/24; D21H 17/28; D21H 17/31; D21H 21/10;** (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **SNF Group**
**42160 Andrézieux-Bouthéon (FR)**

(72) Inventors:
- **RADCHENKO, Alexei**
  **42160 ANDREZIEUX-BOUTHEON (FR)**
- **SOUZY, Renaud**
  **42160 ANDREZIEUX-BOUTHEON (FR)**
- **BRAUN, Olivier**
  **42160 ANDREZIEUX-BOUTHEON (FR)**

(74) Representative: **Ipsilon**
**Le Contemporain**
**50 Chemin de la Bruyère**
**69574 Dardilly Cedex (FR)**

## (54) PROCESS OF PREPARATION OF A FUNCTIONALIZED POLYMER

(57) The present invention relates to a process of preparation of a non-anionic functionalized polymer, said functionalised polymer and its use, in particular in wastewater treatment as a coagulant, or in pulp and paper industry as retention aids, or in specialties such as home and personal care composition as conditioner.

(52) Cooperative Patent Classification (CPC): (Cont.)
**D21H 23/04**

## Description

### Field of the invention

The present invention relates to a process of preparation of a functionalized polymer, said functionalised polymer and its use, in particular in wastewater treatment as a coagulant, or in pulp and paper industry as retention aids, or in specialties such as home and personal care composition as conditioner.

### Prior art

Polysaccharides are natural polymers that are increasingly being used to replace petrochemical-based polymers. They are abundant resources, and they offer the advantage of being renewable and biodegradable. However, they are generally less efficient and have a shorter shelf life than petrochemical-based polymers.

Numerous developments have been made to overcome this lack of performance, notably through functionalization.

Various techniques such as grafting or chemical functionalization are used to improve polysaccharides performances. Generally, the functionalization is carried out via the hydroxyle functions, present on the polysaccharide skeleton, by reaction with an epoxide bearing a quaternary ammonium group, as described in the document M.-P. Labeau, P. Marion, F. Monnet et al., "Chemicals and Fuels from Bio-Based Building Blocks", Wiley, 2016, pp 615-642. Some production processes also deal with nucleophilic substitution on alkyl chlorides. However, this process generally requires hard conditions, reducing the positive impact of using polysaccharides, in addition due to the hard conditions, during these processes the polysaccharide is partially degraded.

Other milder techniques have been developed, for example document WO22254083 describes the reaction of a polysaccharide with a compound having an electron-withdrawing conjugated group by oxa-Michael addition reaction in the presence of a base. By using this process, the functionalization of the polysaccharide is not well controlled. This leads to functionalized polysaccharides having poor applicative performances and moderate biodegradability.

The applicant has developed a functionalization process that is carried out under mild conditions. This novel approach leads to eco-designed functionalized polymers having good applicative performances while maintaining good biodegradability.

The invention is in line with the principle of environmental awareness and the impact of industry and mankind on the planet. The functionalized polymer of the invention has improved performances compared to available functionalized polymers, enabling a reduction of polymer quantity used in the application and thus enabling to have a lower environmental impact.

In addition, the present invention is advantageously carried out using materials of biological origin, such as biomass, or recycled materials. The synthesis of the monomers used in the invention are advantageously a biological synthesis, for example, by enzymatic catalysis. The energy used to carry out the process according to the invention is advantageously derived from a heat pump or of renewable origin, for example wind power, photovoltaics, or, in particular for mobile installations, from fuel cell or lithium battery type.

### Disclosure of the invention

The present invention relates to a process of preparation of a non-anionic functionalized polymer P3 comprising:

(1) providing a polymer P2, having at least one carbon-carbon double bond, previously prepared by reaction Re1 between a polymer P1 and a compound C1 wherein:

(i) the polymer P1 has at least one functional group chosen from: hydroxy(-OH), thiol (-SH), amine (-NH),
(ii) the compound C1 is selected from the group consisting of compounds of formula I, of formula II, of formula III, and mixtures thereof:

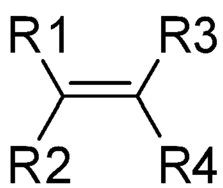 (I) 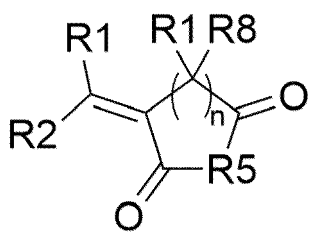 (II) 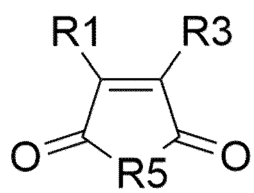 (III),

in which:

- R1, R2 and R3, identical or different, represent a hydrogen atom or a saturated or unsaturated hydrocarbon chain having from 1 to 20 carbon atoms that is linear or branched and may contain one or more heteroatoms selected from the group consisting of oxygen (O), nitrogen (N) or sulfur (S),
- R4 represents a functional group selected from the group consisting of: carboxylic acid (-C(O)OH), a salt of carboxylic acid, anhydride (-C(O)OC(O)R6), acyl halide (-C(O)X in which X is a halogen atom), ester (-C(O) OR7), amide (-C(O)$NH_2$, amide -C(O)N$(R7)_2$, amide
- C(O)NHR7), sulfone (-S($O_2$)OR7), sulfonic acid (-S($O_2$)OH), a salt of sulfonic acid, phosphonate (-P(O) $(OR7)_2$), phosphonic acid (-P(O)$(OH)_2$), a salt of phosphonic acid, hemi-phosphonate (-P(O)(OH)(OR7)), a salt of hemi-phosphonate,
- R5 represents an atom of oxygen (O), nitrogen (N) or sulfur (S),
- n is an integer between 1 and 3,
- R6 is selected from the group consisting of (a) a saturated or unsaturated, linear or branched, hydrocarbon chain having from 1 to 20 carbon atoms, (b) a 5 or 6-membered aryl, and

  (R1)(R2)C=C(R3)-,

  wherein R6 may contain one or more heteroatoms selected from the group consisting of oxygen (O), nitrogen (N), sulfur (S) and mixtures thereof,
- R7 represents a saturated or unsaturated, linear or branched hydrocarbon chain having from 1 to 20 carbon atoms or an aryl, optionally substituted or a glycidyl group,
  wherein R7 can optionally comprise one or several heteroatoms selected from the group consisting of oxygen (O), sulfur (S), nitrogen (N) and mixtures thereof,
- R8 represents a hydrogen atom or a saturated or unsaturated, linear or branched hydrocarbon chain having from 1 to 20 carbon atoms or an optionally substituted aryl,

wherein R8 can optionally comprise one or more heteroatoms selected from the group consisting of oxygen (O), nitrogen (N) or sulfur (S) and mixtures thereof,
(2) preparing polymer P3 by reaction Re2 between the polymer P2 and a compound C2 wherein the compound C2 is selected from the group consisting of compounds of formula (IV) and salts thereof, compounds of formula (V) and salts thereof, and mixtures thereof:

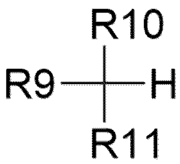
(IV)

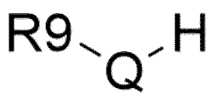
(V)

in which:

- R9, R10 and R11, identical or different, represent, independently from each other, a hydrogen atom; saturated or unsaturated hydrocarbon chain having from 1 to 20 carbon atoms that may contain one or more heteroatoms selected from the group consisting of oxygen (O), nitrogen (N) or sulfur (S) and mixtures thereof, the hydrocarbon groups having from 1 to 20 carbon atoms being optionally substituted with an acid (-C(O)OH) or an ester (-C(O) OR12) function with R12 being a, linear or branched, hydrocarbon chain having from 1 to 20 carbon atoms or an optionally substituted aryl,
  wherein, in formula (IV), at least one of R9, R10 and R11 is an electron-withdrawing group selected from the group consisting of: aldehyde (-CHO), acyl (-COR6), carboxylic acid (-C(O)OH), a salt of carboxylic acid, amide (-C(O) $NH_2$, amide -C(O)N$(R7)_2$, amide - C(O)NHR7), nitrile (-CN), sulfone (-S($O_2$)OR7), sulfonic acid (-S($O_2$)OH), a salt of sulfonic acid, phosphonate (-P(O)$(OR7)_2$), phosphonic acid (-P(O)$(OH)_2$), a salt of phosphonic acid, hemi-phosphonate (-P(O)(OH)(OR7)), a salt of hemi-phosphonate, nitro (-$NO_2$), imine (-CH=N-R7);
- Q represents a sulfur (S), an oxygen (O) atom or N-R10.

The present invention also relates to the non-anionic functionalized polymer P3 obtained according to the functionalization process according to the invention.

The present invention also relates to the use of the non-anionic functionalized polymer P3 in: drilling wells;

cementing wells; oil and/or gas production (enhanced oil recovery, conformance, diversion, hydraulic fracturing) ; water treatment; treatment of fermentation broth; sludge treatment; construction; paper or cardboard manufacture; batteries; wood treatment; treatment of hydraulic compositions (concrete, cement, mortar and aggregates); mining; formulation of cosmetic products; formulation of detergents; textile manufacture; geothermal energy; sanitary napkin manufacturing or in agriculture.

The present invention also relates to the use of the non-anionic functionalized polymer P3 as a flocculant, coagulant, binding agent, draining agent, viscosity-reducing agent, friction-reducing agent, absorbent agent, filler retention agent, conditioning agent, stabilising agent, stabilising agent, film-forming agent, sizing agent, (super)plasticizing agent, dehydration agent, clay inhibitor, thickening agent, or dispersant.

The invention also relates to a process for treating a suspension of solid particles in water or wastewater, comprising contacting said suspension with at least one non-anionic functionalized polymer P3 according to the invention.

The invention also relates to a process for manufacturing a sheet of paper, cardboard or the like, according to which, before the sheet is formed, at least one non-anionic functionalized polymer P3 according to the invention is added to a fiber suspension at one or more injection points.

The present invention relates to a process for conditioning fibers in home and personal care, comprising contacting said fibers with at least one non-anionic functionalized polymer P3 according to the invention, the fibers being advantageously hairs.

## Description of the invention

The non-anionic functionalized polymer P3 is water-soluble or water-swellable. Preferably it is water-soluble.

By "polymer" we mean a homopolymer or a copolymer. A homopolymer is a polymer composed of a single identical repeating unit, while a copolymer is composed of two or more different repeating units.

By "water-soluble polymer", we mean a polymer which gives an aqueous solution without insoluble particles when dissolved with stirring at 25°C at a concentration of 2 g $L^{-1}$, in deionized water, according to regulations (CE) n° 1907/2006.

By "water-swellable polymer", we mean a polymer that can go through gelatinization when exposed to water and heat. This process leads to the dissolution of the polymer in water, increasing the viscosity of the solution by forming a gel-like medium. This generally occurs by hydrolysis of some intramolecular functions on a non-soluble polymer, allowing water molecules to form hydrogen bonds which solubilizes said polymer. This phenomenon is well known, in particular for starch. Water-swellable according to the invention does not refer to superabsorbent polymers which form a three-dimensional network of covalent bonds trapping water molecule and forming a solid. Superabsorbent/hydrogel polymers are therefore excluded from the scope of the invention.

By "esterification or thio-esterification reaction", we mean any chemical reaction where at least one of the products formed is an ester or a thio ester.

By "amidification reaction", we designate any chemical reaction where at least one of the products formed is an amide.

By "Michael addition" reaction, we designate a conjugate nucleophilic addition reaction involving a nucleophile (Michael donors) and a Michael acceptor, oxa-Michael addition, aza-Michael addition and thia-Michael addition are included.

By "X and/or Y" we mean "X," or "Y," or "X and Y."

The invention also covers all possible combinations of the other than disclosed embodiments, whether they are preferred embodiments or given by way of example. Furthermore, when ranges of values are indicated, the limits are part of these ranges. The disclosure also includes all combinations between the limits of these value ranges. For example, the value ranges "1-20, preferably 5-15", imply disclosure of the ranges "1-5", "1-15", "5-20", and "15-20" and the values 1, 5, 15 and 20.

All the particular and/or preferred modes described in the invention are combinable, provided they are not incompatible.

## Process of preparation of a non-anionic functionalized polymer P3

The present invention relates to a process of preparation of a non-anionic functionalized polymer P3 comprising:

(1) providing a polymer P2 having at least one carbon-carbon double bond, previously prepared by reaction Re1 between a polymer P1 and a compound C1 wherein:

(i) the polymer P1 has at least one functional group chosen from: hydroxy(-OH), thiol (-SH), amine (-NH),

(ii) the compound C1 is selected from the group consisting of compounds of formula I, of formula II, of formula III,

and mixtures thereof:

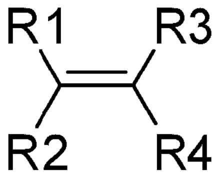 (I) 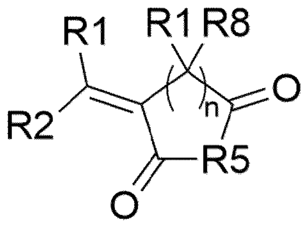 (II) 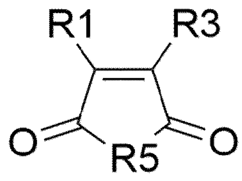 (III),

in which:

- R1, R2 and R3, identical or different, represent a hydrogen atom or a saturated or unsaturated hydrocarbon chain having from 1 to 20 carbon atoms that is linear or branched and may contain one or more heteroatoms selected from the group consisting of oxygen (O), nitrogen (N) or sulfur (S),
- R4 represents a functional group selected from the group consisting of:,, carboxylic acid (-C(O)OH), a salt of carboxylic acid, anhydride (-C(O)OC(O)R6), acyl halide (-C(O)X in which X is a halogen atom), ester (-C(O)OR7), amide (-C(O)$NH_2$, amide -C(O)N$(R7)_2$, amide
- C(O)NHR7),, sulfone (-S($O_2$)OR7), sulfonic acid (-S($O_2$)OH), a salt of sulfonic acid, phosphonate (-P(O)$(OR7)_2$), phosphonic acid (-P(O)$(OH)_2$), a salt of phosphonic acid, hemi-phosphonate (-P(O)(OH)(OR7)), a salt of hemi-phosphonate,,
- R5 represents an atom of oxygen (O), nitrogen (N) or sulfur (S),
- n is an integer between 1 and 3,
- R6 is selected from the group consisting of (a) a saturated or unsaturated, linear or branched, hydrocarbon chain having from 1 to 20 carbon atoms, (b) a 5 or 6-membered aryl, and

  (R1)(R2)C=C(R3)-,

  wherein R6 may contain one or more heteroatoms selected from the group consisting of oxygen (O), nitrogen (N), sulfur (S) and mixtures thereof,
- R7 represents a saturated or unsaturated, linear or branched hydrocarbon chain having from 1 to 20 carbon atoms or an aryl, optionally substituted or a glycidyl group,
  wherein R7 can optionally comprise one or several heteroatoms selected from the group consisting of oxygen (O), sulfur (S), nitrogen (N) and mixtures thereof,
- R8 represents a hydrogen atom or a saturated or unsaturated, linear or branched hydrocarbon chain having from 1 to 20 carbon atoms or an optionally substituted aryl,

wherein R8 can optionally comprise one or more heteroatoms selected from the group consisting of oxygen (O), nitrogen (N) or sulfur (S) and mixtures thereof,
(2) preparing polymer P3 by reaction Re2 between the polymer P2 and a compound C2 wherein the compound C2 is selected from the group consisting of compounds of formula (IV) and salts thereof, compounds of formula (V) and salts thereof, and salts thereof, and mixtures thereof:

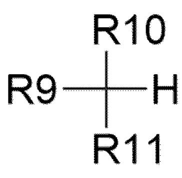 (IV) 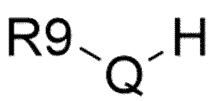 (V)

in which:

- R9, R10 and R11, identical or different, represent, independently from each other, a hydrogen atom; saturated or unsaturated hydrocarbon chain having from 1 to 20 carbon atoms that may contain one or more heteroatoms selected from the group consisting of oxygen (O), nitrogen (N) or sulfur (S) and mixtures thereof, the hydrocarbon groups having from 1 to 20 carbon atoms being optionally substituted with an acid (-C(O)OH) or an ester (-C(O)OR12) function with R12 being a, linear or branched, hydrocarbon chain having from 1 to 20 carbon atoms or an

optionally substituted aryl,

wherein, in formula (IV), at least one of R9, R10 and R11 is an electron-withdrawing group selected from the group consisting of: aldehyde (-CHO), acyl (-COR6), carboxylic acid (-C(O)OH), a salt of carboxylic acid, amide (-C(O)$NH_2$, amide -C(O)N$(R7)_2$, amide - C(O)NHR7), nitrile (-CN), sulfone (-S($O_2$)OR7), sulfonic acid (-S($O_2$)OH), a salt of sulfonic acid, phosphonate (-P(O)$(OR7)_2$), phosphonic acid (-P(O)$(OH)_2$), a salt of phosphonic acid, hemi-phosphonate (-P(O)(OH)(OR7)), a salt of hemi-phosphonate, nitro (-$NO_2$), imine (-CH=N-R7);

- Q represents a sulfur (S), an oxygen (O) atom or N-R10.

By "non-anionic functionalized polymer P3", we mean that the polymer P3 can be cationic, amphoteric, non-ionic. The polymer P3 can comprise anionic functions but cannot contain only anionic functions nor a mixture of only non-ionic and anionic functions, which are thereof excluded from the scope of the invention.

**Step (1) Preparation of polymer P2**

The polymer P2 having at least one carbon-carbon double bond is prepared by reaction Re1 between a polymer P1 and a compound C1 wherein:

(i) the polymer P1 has at least one functional group chosen from: hydroxy(-OH), thiol (-SH), amine (-NH),
(ii) the compound C1 is selected from the group consisting of compounds of formula I, formula II, formula III, and mixtures thereof:

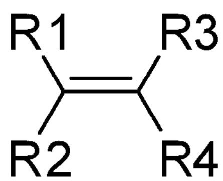 (I) 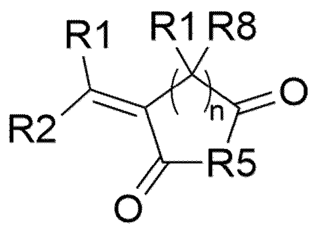 (II) 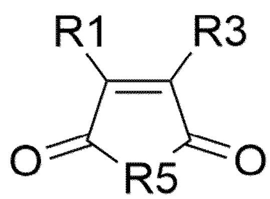 (III),

in which:

- R1, R2 and R3, identical or different, represent a hydrogen atom or a saturated or unsaturated hydrocarbon chain having from 1 to 20 carbon atoms that is linear or branched and may contain one or more heteroatoms selected from the group consisting of oxygen (O), nitrogen (N) or sulfur (S),
- R4 represents a functional group selected from the group consisting of: carboxylic acid (-C(O)OH), a salt of carboxylic acid, anhydride (-C(O)OC(O)R6), acyl halide (-C(O)X in which X is a halogen atom), ester (-C(O)OR7), amide (-C(O)$NH_2$, amide -C(O)N$(R7)_2$, amide
- C(O)NHR7), sulfone (-S($O_2$)OR7), sulfonic acid (-S($O_2$)OH), a salt of sulfonic acid, phosphonate (-P(O)$(OR7)_2$), phosphonic acid (-P(O)$(OH)_2$), a salt of phosphonic acid, hemi-phosphonate (-P(O)(OH)(OR7)), a salt of hemi-phosphonate,
- R5 represents an atom of oxygen (O), nitrogen (N) or sulfur (S),
- n is an integer between 1 and 3,
- R6 is selected from the group consisting of (a) a saturated or unsaturated, linear or branched, hydrocarbon chain having from 1 to 20 carbon atoms, (b) a 5 or 6-membered aryl, and

  (R1)(R2)C=C(R3)-,

  wherein R6 may contain one or more heteroatoms selected from the group consisting of oxygen (O), nitrogen (N), sulfur (S) and mixtures thereof,
- R7 represents a saturated or unsaturated, linear or branched hydrocarbon chain having from 1 to 20 carbon atoms or an aryl, optionally substituted or a glycidyl group,
  wherein R7 can optionally comprise one or several heteroatoms selected from the group consisting of oxygen (O), sulfur (S), nitrogen (N) and mixtures thereof,
- R8 represents a hydrogen atom or a saturated or unsaturated, linear or branched hydrocarbon chain having from 1 to 20 carbon atoms or an optionally substituted aryl,

wherein R8 can optionally comprise one or more heteroatoms selected from the group consisting of oxygen (O), nitrogen

(N) or sulfur (S) and mixtures thereof;

The reaction is advantageously a condensation reaction, preferably chosen from esterification, thio-esterification or amide formation reactions.

The reaction is advantageously carried out in a polar solvent PS1 chosen from: water, isopropanol, tert-butanol, ethanol, acetone, dimethyl sulfoxide, tetrahydrofuran, dimethylformamide and mixture thereof. Preferably it is water.

The reaction is advantageously carried out at a temperature comprised between -10 and 100°C, preferably between 10 and 50°C, more preferably between 15 and 40°C.

The reaction temperature between the polymer P1 and the compound C1 will be adjusted by the skilled man of the art in order to have a polar solvent PS1 in liquid form.

The pH of the reaction is advantageously controlled between 6 and 12, preferably between 7 and 10, more preferably between 7 and 9.

The pH can be adjusted using a base for example an alkali hydroxide or an alkaline earth metal hydroxide or amine base, like sodium hydroxide or potassium hydroxide, sodium methoxide, l,4-diazabicyclo[2.2.2]octane (DABCO), triethylamine (TEA), sodium carbonate ($Na_2CO_3$), pyridine ($C_5H_5N$), sodium bicarbonate ($NaHCO_3$), potassium carbonate ($K_2CO_3$), potassium bicarbonate ($KHCO_3$), sodium ethoxide, and. potassium tert-butoxide (tBuOK), diazabicyclo undecene (DBU), sodium methoxide or a mixture thereof. Preferably, the base is sodium hydroxide.

The reaction lasts advantageously between 1 minute and 1500 minutes, preferably between 30 minutes and 240 minutes, more preferably between 60 minutes and 120 minutes.

The reaction is advantageously carried out at atmospheric pressure.

The polymer P2 is prepared by reaction between a polymer P1 having at least one functional group chosen from hydroxy(-OH), thiol (-SH), amine (-NH) and a compound C1 selected from the group consisting of compounds of formula I, formula II, formula III, and mixtures thereof as described above.

The polymer P1 can be water-soluble or water-swellable. Preferably it is water-soluble.

The polymer P1 can be a natural polymer or a synthetic polymer. Preferably it is a natural polymer.

The natural polymer can be a saccharide or a protein. Preferably it is a saccharide.

By "saccharide" we mean a saccharide, a polysaccharide as well as a modified polysaccharide.

By "modified polysaccharide" we mean a polysaccharide that has undergone one or more treatments, which may be physical and/or chemical and/or enzymatic.

By "protein" we mean a protein as well as a modified protein.

By "modified protein" we mean a protein that has undergone one or more treatments, which may be physical and/or chemical and/or enzymatic.

The polysaccharide can be extracted from plants, animals or produced by micro-organisms such as bacteria, fungi, prokaryotes and eukaryotes. For example, xanthan gum can be produced by Xanthomonas campestris, gellan by Sphingomonas paucimobllis, xyloglucan can be extracted from tamarind seeds.

Preferably, the polysaccharide is selected from the group consisting of: starch, dextrin, maltodextrin, tamarind gum (preferably consisting of xyloglucan), guar gum, locust bean gum (preferably consisting of galactomannan), carboxymethyl cellulose, pectin, and other industrial gums and polymers such as tara gum, fenugreek, aloe vera, chia seeds, linseeds, psyllium seeds, quince seeds, xanthan gum, gellan gum, welan gum, rhamsan gum, dextran, curdlan, pullulan, scleroglucan, schizophyllan, chitin, hydroxyalkylcellulose, arabin (preferably obtained from sugar beet), arabinoxylane, curdlan, galactans (preferably obtained from lupins and/or potatoes), pectic galactans (preferably obtained from potatoes), galactomannan (preferably obtained from carob), glucomannan, lichenan (preferably obtained from Iceland moss), mannan (preferably obtained from ivory nut), pachyman, rhamnogalacturonan, acacia gum, agar, alginates, carrageenans, chitosan, clavans, hyaluronic acid, heparin, inulin, cellodextrins, cellulose, cellulose derivatives and mixtures thereof.

According to a preferred embodiment, the natural polymer can undergo chemical grafting reactions with various synthetic substituents, for example, carboxyalkyl starch, like carboxymethyl starch, carboxyalkyl cellulose, like carboxymethyl cellulose, carboxyalkyl guar, like carboxymethyl guar, hydroxypropyl methylcellulose, carboxyalkyl hydroxyalkyl cellulose, like carboxymethyl hydroxypropyl cellulose, carboxyalkyl hydroxyalkyl guars, like carboxymethyl hydroxypropyl guar. This type of reaction is familiar to those skilled in the art as classic chemical reactions applied to natural polymers.

According to a preferred embodiment, the polysaccharide, preferably chosen from: starch and/or its derivatives such as carboxymethyl starch; cellulose and/or its derivatives such as carboxymethyl cellulose, hemicellulose; polysaccharides extracted from algae such as agarose, carrageenans, alginates; pectins; inulin; guar gums; konjac gums; dextran; pullulan; gellan gum; curdlane; xanthan gum; maltodextrine.

The protein may come from an animal, a vegetable, a bacterium, or be derived from a fungus, an algae or a yeast.

Animal proteins include milk proteins such as β-lactoglobulin, casein and whey; serum proteins such as horse serum; fibrous dermal proteins such as collagen, elastin and silk proteins.

Vegetable proteins include proteins extracted from corn, wheat, barley, oats, soya or peas, such as glutelin, prolamin, zein and gluten. Advantageously, vegetable proteins are chosen from soy, wheat, oat or pea proteins. Proteins

can also be obtained from seeds, e.g. soy, cotton, peanut, sunflower, rapeseed, coconut, linseed, sesame, peas, beans or lentils.

Bacterial proteins include *Pseudomonas*, *Lactobacillus* and *E.Coli.* Fungi proteins include *Penicillium*, and algae proteins include blue-green algae, green algae such as *Chlorella* and *Spirulina.*

In a preferred mode, the protein is chosen from casein, serum proteins or wheat proteins.

Without being limited, the synthetic polymer may be selected from, for example, polyvinyl alcohol or their copolymers, polyallyl alcohol or their copolymers, polyvinylamine or their copolymers, polyallyl amine or their copolymers, polyethylenimine and mixture thereof.

In a preferred embodiment, the polymer P1 has an average molecular weight ($M_n$) (determined by gel permeation chromatography) comprised between 300 and 30 000 000 g/mol, preferably between 1 000 and 1 000 000 g/mol, more preferably between 5 000 and 500 000 g/mol.

The analytical conditions are as follows:

- 1 Shodex pre-column referenced SB807-G
- 2 Shodex OHpak columns mounted in series, referenced SB-807 HQ / SB-805 HQ)
- The columns are coupled with a refractive index detector referenced Optilab T-rEX and Dawn Heleos II 18 angles marketed by the company Wyatt Technology.

In a preferred embodiment, the polymer P1 is a natural polymer, more preferably a polysaccharide.

The compound C1 is an $\alpha,\beta$-unsaturated Michael acceptor that reacts with polymer P1 having at least one functional group chosen from chosen from hydroxy(-OH), thiol (-SH), amine (-NH) .

The compound C1 can be anionic, cationic, amphoteric, non-ionic, zwitterionic or hydrophobic. Preferably it is non-ionic.

The compound C1 is selected from the group consisting of compounds of formula I, of formula II, of formula III, and mixtures thereof:

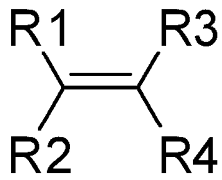 (I) 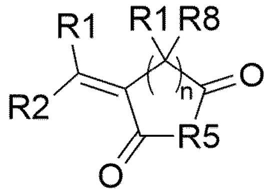 (II) 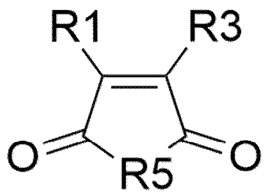 (III);

in which:

- R1, R2 and R3, identical or different, represent a hydrogen atom or a saturated or unsaturated hydrocarbon chain having from 1 to 20 carbon atoms that is linear or branched and may contain one or more heteroatoms selected from the group consisting of oxygen (O), nitrogen (N) or sulfur (S),
- R4 represents a functional group selected from the group consisting of: carboxylic acid (-C(O)OH), a salt of carboxylic acid, anhydride (-C(O)OC(O)R6), acyl halide (-C(O)X in which X is a halogen atom), ester (-C(O)OR7), amide (-C(O)$NH_2$, amide -C(O)N$(R7)_2$, amide
- C(O)NHR7), sulfone (-S($O_2$)OR7), sulfonic acid (-S($O_2$)OH), a salt of sulfonic acid, phosphonate (-P(O)$(OR7)_2$), phosphonic acid (-P(O)$(OH)_2$), a salt of phosphonic acid, hemi-phosphonate (-P(O)(OH)(OR7)), a salt of hemi-phosphonate,
- R5 represents an atom of oxygen (O), nitrogen (N) or sulfur (S),
- n is an integer between 1 and 3,
- R6 is selected from the group consisting of (a) a saturated or unsaturated, linear or branched, hydrocarbon chain having from 1 to 20 carbon atoms, (b) a 5 or 6-membered aryl, and (R1)(R2)C=C(R3)-,
  wherein R6 may contain one or more heteroatoms selected from the group consisting of oxygen (O), nitrogen (N), sulfur (S) and mixtures thereof,
- R7 represents a saturated or unsaturated, linear or branched hydrocarbon chain having from 1 to 20 carbon atoms or an aryl, optionally substituted or a glycidyl group,
  wherein R7 can optionally comprise one or several heteroatoms selected from the group consisting of oxygen (O), sulfur (S), nitrogen (N) and mixtures thereof,
- R8 represents a hydrogen atom or a saturated or unsaturated, linear or branched hydrocarbon chain having from 1 to 20 carbon atoms or an optionally substituted aryl,

wherein R8 can optionally comprise one or more heteroatoms selected from the group consisting of oxygen (O), nitrogen (N) or sulfur (S) and mixtures thereof.

The compound C1 is preferably chosen from: methacrylic anhydride, itaconic anhydride, maleic anhydride, acryloyl chloride, acryloyl bromide, methacryloyl chloride, methacryloyl bromide or glycidyl (meth)acrylate.

The molar ratio between the compound C1 and the functional groups of the polymer P1 is advantageously comprised between 2:1 and 1:10000, preferably between 1:1 and 1:1000, more preferably between 1:3 and 1:100.

When the compound C1 is hydrophobic, the molar ratio between polymer P1 and compound C1 is adjusted in order to obtain a polymer P2 which is water-soluble.

The degree of substitution (DS) of the polymer P1 by the compound C1 is advantageously comprised between 0.00001 and 1, preferably between 0.05 and 1, more preferably between 0.1 and 0.5.

By "degree of substitution" we mean the average amount of functional groups on the polymer P1 that reacted with the compound C1 compared to the total number of unreacted functional groups on the polymer P1 before the reaction. The maximum value is therefore 1, in this case, all the functional groups on the polymer P1 have reacted with compound C1.

The degree of substitution is determined by 1 H NMR spectroscopy (Bruker 400MHz, equipped with multinuclear probe).

**Step (2) Reaction between the polymer P2 and the compound C2 to form the non-anionic functionalized polymer P3**

Compound C2 is selected from the group consisting of compounds of formula (IV) and salts thereof, compounds of formula (V) and salts thereof, and mixtures thereof:

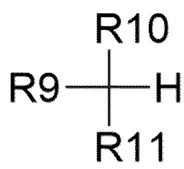 (IV)

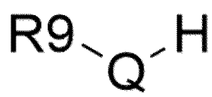 (V)

in which:

- R9, R10 and R11, identical or different, represent, independently from each other, a hydrogen atom; saturated or unsaturated hydrocarbon chain having from 1 to 20 carbon atoms that may contain one or more heteroatoms selected from the group consisting of oxygen (O), nitrogen (N) or sulfur (S) and mixtures thereof, the hydrocarbon groups having from 1 to 20 carbon atoms being optionally substituted with an acid (-C(O)OH) or an ester (-C(O)OR12) function with R12 being a, linear or branched, hydrocarbon chain having from 1 to 20 carbon atoms or an optionally substituted aryl,
  wherein, in formula (IV), at least one of R9, R10 and R11 is an electron-withdrawing group selected from the group consisting of: aldehyde (-CHO), acyl (-COR6), carboxylic acid (-C(O)OH), a salt of carboxylic acid, amide (-C(O)$NH_2$, amide -C(O)N$(R7)_2$, amide - C(O)NHR7), nitrile (-CN), sulfone (-S($O_2$)OR7), sulfonic acid (-S($O_2$)OH), a salt of sulfonic acid, phosphonate (-P(O)$(OR7)_2$), phosphonic acid (-P(O)$(OH)_2$), a salt of phosphonic acid, hemi-phosphonate (-P(O)(OH)(OR7)), a salt of hemi-phosphonate, nitro (-$NO_2$), imine (-CH=N-R7);
- Q represents a sulfur (S), an oxygen (O) atom or N-R10.

The compound C2 can have an anionic, a non-ionic, or cationic charge. Preferably, in conditions of use, C2 has cationic units.

Preferably the compound C2 is of formula (V) wherein Q represents a sulfur (S).

In another preferred embodiment, the compound C2 is of formula (V) wherein Q represents an amine in neutral form.

The compound C2 can comprise more than one hydroxy group and/or thiol group and/or amino group.

Preferably, the compound C2 is of formula (V) wherein Q represents a sulfur and R9 is a hydrocarbon chain having from 1 to 20 carbon atoms that may contain one or more heteroatoms selected from the group consisting of oxygen (O), nitrogen (N) or sulfur (S).

In a preferred embodiment, the compound C2 is of formula (V) wherein Q represents a sulfur and contains an amine or ammonium function or an hydroxide.

In a particular embodiment, the compound C2 is selected from the group consisting of amino alcohols, amino thiols or amino acids and salts thereof.

The compound C2 advantageously comprises at most 3 thiol groups, preferably at most 2 thiol groups, more

preferably 1 thiol group.

When the compound C2 comprises more than 1 thiol group, its quantity is adjusted in order to prevent the formation of a three-dimensional network in order to obtain a functionalized polymer which is water-soluble or water-swellable and not a three-dimensional network of covalent bonds.

In a preferred embodiment, the compound C2 is chosen from:

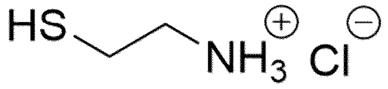 (VII) 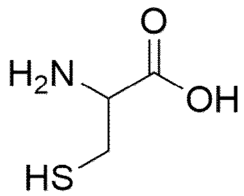 (VIII) 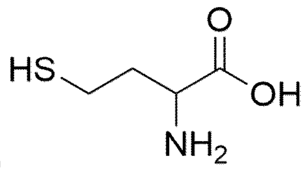 (IX)

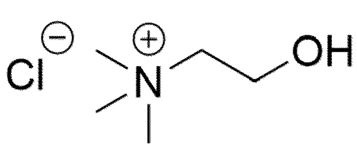 (X) 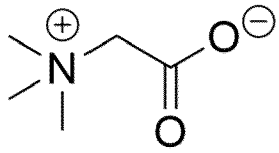 (XI)

The reaction between the polymer P2 and the compound C2 is a Michael addition reaction, wherein the polymer P2 is the Michael acceptor and the compound C2 plays the role of Michael donor. They react to form the non-anionic functionalized polymer P3 by a nucleophilic addition of the Michael donor on the Michael acceptor to form a Michael adduct corresponding to the non-anionic functionalized polymer P3.

The molar ratio between the polymer P2 and the compound C2 is advantageously comprised between 1:2 and 10:1, preferably between 1:1 and 2:1, more preferably is 1:1.

When the compound C2 is hydrophobic, the molar ratio between the polymer P2 and the compound C2 is adjusted in order to obtain a non-anionic functionalized polymer P3 which is water-soluble or water-swellable.

The reaction between the polymer P2 and the compound C2 is advantageously carried out in a polar solvent PS2 chosen from: water, isopropanol, acetone, dimethyl sulfoxide, tetrahydrofuran, dimethylformamide and mixture thereof. Preferably it is water.

The reaction between the polymer P2 and the compound C2 is advantageously carried out at a temperature comprised between -5 and 100°C, preferably between 10 and 60°C, more preferably between 15 and 30°C.

The reaction temperature during the reaction between the polymer P2 and the compound C2 will be adjusted by the skilled man of the art in order to have a polar solvent PS2 in liquid form.

The reaction between the polymer P2 and the compound C2 is advantageously adjusted to a pH comprised between 6 and 12, preferably between 6.5 and 10, more preferably between 7 and 9.

The reaction between the polymer P2 and the compound C2 lasts advantageously between 10 minutes and 3 000 minutes, preferably between 60 minutes and 600 minutes, more preferably between 60 minutes and 400 minutes.

The reaction between the polymer P2 and the compound C2 is advantageously carried out at atmospheric pressure.

The non-anionic functionalized water-polymer P3 can be a non-ionic, a cationic, an amphoteric or a zwitterionic polymer depending on the choice of compound C1 and C2, and/or subsequent optionally quaternarization or hydrolysis reaction on the non-anionic functionalized polymer P3.

In a particular embodiment, the non-anionic functionalized polymer P3 is quaternized.

Those skilled in the art will know how to prepare quaternized functionalized polymer, for example using a quaternizing agent of the R-X type, where R is an alkyl group and X is a halogen or sulfate.

The term "quaternizing agent" refers to a molecule capable of alkylating a tertiary amine.

The quaternizing agent may be chosen from dialkyl sulfates containing from 1 to 6 carbon atoms or alkyl halides containing from 1 to 6 carbon atoms. Preferably, the quaternizing agent is chosen from methyl chloride, benzyl chloride, dimethyl sulfate or diethyl sulfate.

In a particular embodiment the non-anionic functionalized polymer P3 is washed to remove undesired salts, side products and unreacted reagents.

In a preferred embodiment, compound C1 and compound C2 are chosen from at least partially renewable and non-fossil sources.

In the context of the invention, the terms "renewable and non-fossil" are used to designate the origin of a chemical compound derived from biomass or from synthesis gas (syngas), i.e. resulting from one or more chemical transformations

carried out on one or more natural and non-fossil raw materials. The terms "bio-sourced" or "bio-resourced" can also be used to characterize the renewable and non-fossil origin of a chemical compound. The renewable and non-fossil origin of a compound includes renewable and non-fossil raw materials stemming from the circular economy, which have been previously recycled, once or several times, in a biomass material recycling process, such as materials from polymer depolymerization or pyrolysis oil processing.

According to the invention, the "at least partially renewable and non-fossil" quality of a compound means a bio-sourced carbon content preferably between 5wt% and 100wt% relative to the total carbon weight of said compound.

In the context of the invention, the ASTM D6866-21 standard Method B is used to characterize the bio-sourced nature of a chemical compound and to determine the bio-sourced carbon content of said compound. The value is expressed as a weight percentage (wt%) of bio-sourced carbon relative to the total carbon weight in said compound.

The ASTM D6866-21 standard is a test method that teaches how to experimentally measure the bio-sourced carbon content of solids, liquids and gaseous samples by radiocarbon analysis.

This standard primarily uses Accelerator Mass Spectrometry (AMS) technology. This technique is used to naturally measure the radionuclides present in a sample, wherein the atoms are ionized, then accelerated to high energies, then separated, and individually counted in Faraday cups. This high-energy separation is extremely effective at filtering out isobaric interference, so that AMS is able to accurately measure abundances of carbon-14 relative to carbon-12 (14C/12C) to an accuracy of $1.10^{-15}$.

The ASTM D6866-21 standard Method B uses AMS and IRMS (Isotope Ratio Mass Spectroscopy). The test method allows to directly differentiate contemporary carbon-based carbon atoms from fossil-based carbon atoms. A measure of the carbon-14 to carbon-12 or carbon-14 to carbon-13 content of a product is determined against a modern carbon-based reference material accepted by the radiocarbon dating community such as the NIST's Standard Reference Material (SRM) 4990C (oxalic acid).

The sample preparation method is described in the standard and does not require any special comment as it is a commonly used procedure.

Analysis, interpretation and reporting of results are described below. Isotope ratios of carbon-14 to carbon-12 content or carbon-14 to carbon-13 content are measured using AMS. Isotope ratios of carbon-14 to carbon-12 content or carbon-14 to carbon-13 content are determined relative to a standard traceable via the NIST SRM 4990C modern reference standard. The "fraction of modern" (fM) represents the amount of carbon-14 in the tested product relative to the modern standard. It is most often referred to as percent modern carbon (pMC), the percentage equivalent to fM (e.g. fM 1 = 100 pMC).

All pMC values obtained from radiocarbon analyses must be corrected for isotopic fractionation using a given stable isotope. The correction should be made using the carbon-14 to carbon-13 values determined directly using the AMS where possible. If this is not possible, the correction should be made using the delta 13C ($\delta$13C) measured by IRMS, CRDS (Cavity Ring Down Spectroscopy) or any other equivalent technology that can provide accuracy to within plus or minus 0.3 per thousand.

"Zero pMC" represents the total absence of measurable 14C in a material above the background signals, thus indicating a fossil (e.g. petroleum-based) carbon source. A value of 100 pMC indicates a fully "modern" carbon source. A pMC value between 0 and 100 indicates a proportion of carbon derived from a fossil source relative to a "modern" source.

The pMC may be higher than 100% due to the persistent, but diminishing, effects of 14C injection into the atmosphere caused by atmospheric nuclear testing programmes. The pMC values need to be adjusted by an atmospheric correction factor (REF) to obtain the actual bio-sourced content of the sample.

The correction factor is based on the excess 14C activity in the atmosphere at the time of testing. A REF value of 102 pMC was determined for 2015 based on $CO_2$ measurements in the air in a rural area of the Netherlands (Lutjewad, Groningen). The first version of this standard (ASTM D6866-04) in 2004 had referenced a value of 107.5 pMC, while the later version ASTM D6866-10 (2010) had referenced a value of 105 pMC. These data points represent a drop of 0.5 pMC per year. Consequently, on 2 January of each year, the values in Table 1 below were used as REF value until 2019, reflecting the same decrease of 0.5 pMC per year. The REF values (pMC) for 2020 and 2021 have been determined to be 100.0 based on continuous measurements in the Netherlands (Lutjewad, Groningen) until 2019. References for reporting carbon isotope ratio data are provided below for 14C and 13C, respectively Roessler, N., Valenta, R. J., and van Cauter, S., "Time-resolved Liquid Scintillation Counting", Liquid Scintillation Counting and Organic Scintillators, Ross, H., Noakes, J. E., and Spaulding, J. D., Eds., Lewis Publishers, Chelsea, MI, 1991, pp. 501-511. Allison, C. E., Francy, R. J., and Meijer, H. A. J., "Reference and Intercomparison Materials for Stable Isotopes of Light Elements", International Atomic Energy Agency, Vienna, Austria, IAEATECHDOC- 825, 1995.

The percentage of the bio-sourced carbon content is calculated by dividing pMC by REF and multiplying the result by 100. For example, [102 (pMC) / 102 (REF)] $\times$ 100 = 100% bio-sourced carbon. The results are indicated as a weight percentage (wt%) of bio-sourced carbon relative to the total carbon weight in said compound.

Table 1: Reference of percentage of modern carbon (pMC)

| REF Year | pMC |
|---|---|
| 2015 | 102.0 |
| 2016 | 101.5 |
| 2017 | 101.0 |
| 2018 | 100.5 |
| 2019 | 100.0 |
| 2020 | 100.0 |
| 2021 | 100.0 |
| 2022 | 100.0 |
| 2023 | 100.0 |

In a particularly preferred embodiment, compound C1 and compound C2 are 100 wt% renewable and non-fossil source.

The present invention also relates to the non-anionic functionalized polymer P3 obtained according to the process of the invention.

The present invention also relates to the use of the non-anionic functionalized polymer P3 in: drilling wells; cementing wells; oil and/or gas production (enhanced oil recovery, conformance, diversion, hydraulic fracturing); water treatment; treatment of fermentation broth; sludge treatment; construction; paper or cardboard manufacture; batteries; wood treatment; treatment of hydraulic compositions (concrete, cement, mortar and aggregates); mining; formulation of cosmetic products; formulation of detergents; textile manufacture; geothermal energy; sanitary napkin manufacturing or in agriculture.

The present invention also relates to the use of the non-anionic functionalized polymer P3 as a flocculant, coagulant, binding agent, draining agent, viscosity-reducing agent, friction-reducing agent, absorbent agent, filler retention agent, conditioning agent, stabilising agent, stabilising agent, film-forming agent, sizing agent, (super)plasticizing agent, dehydration agent, clay inhibitor, thickening agent, or dispersant. The invention also relates to a process for treating a suspension of solid particles in water or wastewater, comprising contacting said suspension with at least one non-anionic functionalized polymer P3 according to the invention.

The invention also relates to a process for manufacturing a sheet of paper, cardboard or the like, according to which, before a sheet is formed, at least one non-anionic functionalized polymer P3 according to the invention is added to a fiber suspension at one or more injection points.

The present invention relates to a process for conditioning fibers in home and personal care, comprising contacting said fibers with at least one non-anionic functionalized polymer P3 according to the invention, the fibers being advantageously hairs.

## Examples

The following examples illustrate the invention without limiting its scope.

*Starting materials*

- Starch were provided by:

    * Starch-1: Roquette Frères, commercial name Stabilys® A053;
    * Starch-2: Roquette Frères, commercial name Stabilys® A020.

- Xanthan gums were provided by:

    * Xanthan gum-1: Jungbunzlauer, commercial name Xanthan Gum FFPC;
    * Xanthan gum-2: CP Kelco, commercial name KELZAN® AP-AS.

- Maltodexrins were provided by:

  * Maltodextrin-1: Tate & Lyle, commercial name Maltosweet® 180;
  * Maltodextrin-2: Prodechim, commercial name MALTODEXTRINE 18DE;
  * Maltodextrin-3: Cargill, commercial name C-Dry™ MD 01958.

- Methacrylic anhydride was provided by Evonik Operations GmbH, commercial name VISIOMER® MAAH;
- Itaconic anhydride was provided by TCI.
- Maleic anhydride was provided by Sigma-Aldrich.
- Cysteamine was provided by ABCR in the form of its hydrochloride salt.
- L-Cysteine was provided by Sigma-Aldrich
- Polyquaternium 10 (PQ10)

*Analysis*

Final and intermediate products were analyzed by 1H NMR spectroscopy using Bruker 400MHz spectrometer equipped with multinuclear probe.

The transmittance values were measured with a UV-photometer DR 3900 (HACH). Transmittance is the percentage of light transmitted through a 1 cm thick sample in a quartz cell at a wavelength of 600 nm.

The hair combing was tested with Dia-Stron equipped with the system of data treatment.

The bulk viscosity, measured with a Brookfield viscosimeter LV3 module; speed 60 rpm; at 25°C.

**Procedure for the preparation of functionalized polymers P3-1 to P3-20**

All reactions were conducted at room temperature (19 - 23°C) without heating or cooling.

Process of preparation of non-anionic functionalized polymer **P3-1**

*Preparation of polymer **P2***

210 g of water was introduced into a 1 L reactor equipped with a mechanical stirrer and a pH-meter.

210 g of Starch-1 **(P1)** was added slowly and mixed at 500 rpm throughout the reaction.

The pH was adjusted to 8 with a 50% NaOH aqueous solution.

The pH was maintained between 7 and 9 during the reaction.

2 g of methacrylic anhydride **(Compound C1 of formula III**) were added dropwise during 1 h. At the end of the reaction, the polymer **P2** was obtained.

The degree of substitution (DS) was measured by $^1$H NMR to be 0.003.

*Preparation process for the synthesis of non-anionic functionalized polymer **P3-1***

1.92 g of cysteamine hydrochloride **(Compound C2 of formula V)** was added to the polymer **P2.** The reaction mixture was stirred for 2 h to give the **non-anionic functionalized polymer P3-1.**

Functionalized polymers **P3-2** to **P3-20** were synthetized according to the process described for polymer **P3-1.**

Their composition and nature are presented in Table 2.

Polymers obtained with the functionalization process according to the invention are compared to polyquaternium-10 (PQ10), a quaternized hydroxyethyl cellulose. PQ10 is used as reference as this compound represents the most and best functionalized biopolymer available on the market.

Table 2: Composition and nature of functionalized polymers **P3-1** to **P3-20**

| Functionalized polymers P3 | P1 | C1 | DS of P2 | C2 | Molar ratio C1:C2 |
|---|---|---|---|---|---|
| P3-1 | Starch-1 | Methacrylic anhydride | 0.003 | Cysteamine | 1:1.3 |
| P3-2 | Starch-1 | Methacrylic anhydride | 0.033 | Cysteamine | 1:1.3 |
| P3-3 | Starch-1 | Methacrylic anhydride | 0.167 | Cysteamine | 1:1.3 |
| P3-4 | Starch-1 | Methacrylic anhydride | 0.333 | Cysteamine | 1:1.3 |

(continued)

| Functionalized polymers P3 | P1 | C1 | DS of P2 | C2 | Molar ratio C1:C2 |
|---|---|---|---|---|---|
| P3-5 | Starch-1 | Maleic anhydride | 0.167 | Cysteamine | 1:1.3 |
| P3-6 | Starch-1 | Itaconic anhydride | 0.167 | Cysteamine | 1:1.3 |
| P3-7 | Starch-2 | Maleic anhydride | 0.167 | Cysteamine | 1:1.3 |
| P3-8 | Starch-2 | Methacrylic anhydride | 0.167 | Cysteamine | 1:1.3 |
| P3-9 | Maltodextrin-1 | Methacrylic anhydride | 0.033 | Cysteamine | 1:1.3 |
| P3-10 | Maltodextrin-1 | Methacrylic anhydride | 0.167 | Cysteamine | 1:1.3 |
| P3-11 | Maltodextrin-2 | Methacrylic anhydride | 0.167 | Cysteamine | 1:1.3 |
| P3-12 | Maltodextrin-3 | Methacrylic anhydride | 0.167 | Cysteamine | 1:1.3 |
| P3-13 | Maltodextrin-1 | Maleic anhydride | 0.033 | Cysteamine | 1:1.3 |
| P3-14 | Xanthan gum-2 | Methacrylic anhydride | 0.033 | Cysteamine | 1:1.3 |
| P3-15 | Xanthan gum-1 | Methacrylic anhydride | 0.033 | Cysteamine | 1:1.3 |
| P3-16 | Xanthan gum-2 | Methacrylic anhydride | 0.167 | Cysteamine | 1:1.3 |
| P3-17 | Xanthan gum-2 | Itaconic anhydride | 0.033 | Cysteamine | 1:1.3 |
| P3-18 | Starch-1 | Methacrylic anhydride | 0.003 | Cysteine | 1:1.3 |
| P3-19 | Starch-1 | Methacrylic anhydride | 0.033 | Cysteine | 1:1.3 |
| P3-20 | Starch-1 | Methacrylic anhydride | 0.167 | Cysteine | 1:1.3 |

***Example 1*** - ***Biodegradation tests***

The biodegradability of the polymers was evaluated according to OECD 301F procedure. The results are presented as the oxygen consumption relative to the theoretical oxygen demand (ThOD).

A polymer is considered as inherently biodegradable if the biodegradability reaches between 20 and 60% in 28 days according to OECD 301F.

A polymer is considered as readily biodegradable if the biodegradability reaches at least 60% in 28 days according to OECD 301F.

A polymer is considered as ultimately biodegradable if the biodegradability reaches at least 60% in 90 days according to OECD 301.

The results are summarized in Table 3.

Table 3: Biodegradability of polymers **P3-1** to **P3-20**

| Functionalized polymers | Biodegradability test at 28 days (%) | Biodegradability test at 60 days (%) |
|---|---|---|
| P3-1 | 88.5 | 91.6 |
| P3-2 | 73.4 | 76.6 |
| P3-3 | 54.7 | 67.7 |
| P3-4 | 47.8 | 62.9 |
| P3-5 | 51.7 | 65.4 |
| P3-6 | 53.2 | 69.5 |
| P3-7 | 51.6 | 61.5 |
| P3-8 | 54.1 | 70.9 |
| P3-9 | 77.3 | 81.3 |
| P3-10 | 62.6 | 72.2 |
| P3-11 | 61.3 | 71.6 |

(continued)

| Functionalized polymers | Biodegradability test at 28 days (%) | Biodegradability test at 60 days (%) |
|---|---|---|
| P3-12 | 63.7 | 70.4 |
| P3-13 | 69.7 | 74.2 |
| P3-14 | 42.3 | 92.3 |
| P3-15 | 46.7 | 61.5 |
| P3-16 | 42.2 | 63.3 |
| P3-17 | 47.3 | 63.5 |
| P3-18 | 90.3 | 91.9 |
| P3-19 | 85.5 | 89.3 |
| P3-20 | 60.6 | 68.8 |

All the polymers synthesized according to the process of the invention show a good biodegradability at 28 days and are almost completely biodegraded after 60 days.

**Application tests**

***Example 2 - Wastewater treatment***

A coagulation test was performed with synthetic river water (W1) (Kaolin 0.5 g/L, humic acid 0.015 g/L) and with the suspension of solid from Allinges quarries (W2) (100 g/L).

After the addition of a coagulant mixture was stirred at 277 rpm for 1 minute, then at 32 rpm for 5 minutes.

The stirring was stopped after 15 minutes and the turbidity was measured using a portable turbidimeter 2100Q (HACH).

The turbidity results are presented in Table 4.

Table 4: Comparison of turbidity between functionalized polymers **P3-3, P3-5, P3-8, P3-12, P3-16** and **P3-20** according to the invention and **Starch-1** and **PQ10.**

| Trial | Polymer | Water | Polymer dosage (ppm) | Turbidity (NTU) |
|---|---|---|---|---|
| 1 | - | W1 | - | 534 |
| 2 | Starch-1 | W1 | 20 | 546 |
| 3 | P3-3 | W1 | 20 | 22 |
| 4 | P3-5 | W1 | 20 | 12 |
| 5 | P3-8 | W1 | 20 | 15 |
| 6 | P3-12 | W1 | 20 | 16 |
| 7 | P3-16 | W1 | 20 | 19 |
| 8 | P3-20 | W1 | 20 | 14 |
| 9 | PQ10 | W1 | 20 | 222 |
| 10 | - | W2 | - | 634 |
| 11 | Starch-1 | W2 | 20 | 623 |
| 12 | P3-3 | W2 | 20 | 21 |
| 13 | P3-5 | W2 | 20 | 14 |
| 14 | P3-8 | W2 | 20 | 17 |
| 15 | P3-12 | W2 | 20 | 13 |
| 16 | P3-16 | W2 | 20 | 16 |
| 17 | P3-20 | W2 | 20 | 114 |

(continued)

| Trial | Polymer | Water | Polymer dosage (ppm) | Turbidity (NTU) |
|---|---|---|---|---|
| 18 | PQ10 | W2 | 20 | 247 |

These results demonstrate that non-modified biopolymers such as **starch** do not have any coagulating activity while modified biopolymers from the prior art like **PQ10** have limited performances compared to new modified biopolymers according to the invention.

***Example 3 - Home and personal care***

The following shampoo was formulated:

- Sodium lauryl ether sulfate (11 wt%);
- Cocobetaine (2 wt%);
- Functionalized polymer (0.3 wt%);
- Deionized water (q.s.f. 100%)

The bulk viscosity was adjusted with NaCl in order to obtain a final viscosity between 8000 and 12000 cPs.
The transmittance, the foam appearance, the stability and the combing are measured. Theresults are presented in Table 5:

Table 5: Characteristics of the polymers **P3-3, P3-5, P3-8, P3-12, P3-16** and **P3-20** according to the invention of the shampoo in comparison to **PQ10.**

| Trial | Reference | Bulk viscosity (cPs) | Transmittance at 600 nm (%) | Foam appearance and stability | Combing (0=very bad, 10=very good) |
|---|---|---|---|---|---|
| 19 | P3-3 | 10260 | 95.6 | rich smooth foam that maintains during time | 8 |
| 20 | P3-5 | 10210 | 93.4 | rich smooth foam that maintains during time | 9 |
| 21 | P3-8 | 10430 | 89.9 | rich smooth foam that maintains during time | 7 |
| 22 | P3-12 | 10230 | 90.6 | rich smooth foam that maintains during time | 8 |
| 23 | P3-16 | 10600 | 91.4 | rich smooth foam that maintains during time | 8 |
| 24 | P3-20 | 10050 | 91.9 | rich smooth foam that maintains during time | 8 |
| 25 | PQ10 | 10020 | 89.5 | acceptable foam that does not maintain during time | 5 |

The properties induced by the functionalized polymers according to the invention in the shampoo formulation are better than the properties of**PQ10**.

***Example 4 - Paper industry***

The properties of Filler Retention (FR) of the functionalized polymers of the invention were evaluated. The higher the values, the better the performance. For the test procedure for evaluating the filler retention, the various results were obtained using a Britt Jar, with a stirring rate of 1000 rpm.
The % FR is calculated by the following formula:

%FR= 100 × (Headbox ash consistency - White water ash consistency) / (Headbox ash consistency).

The polymers performances are compared to a blank.

The results are presented in Table 6.

Table 6: Comparison of the Filler Retention results between the polymers **P3-3, P3-5, P3-8, P3-12, P3-16** and **P3-20** of the invention and **PQ10.**

| Trial | Polymer | FR (%) |
|---|---|---|
| 26 | P3-3 | 24.9 |
| 27 | P3-5 | 22.2 |
| 28 | P3-8 | 24.7 |
| 29 | P3-12 | 23.9 |
| 30 | P3-16 | 25.2 |
| 31 | P3-20 | 24.1 |
| 32 | PQ10 | 16.3 |

The results demonstrate that the biopolymers of the invention display good performances in filler retention compared to **PQ10.**

**Claims**

**1.** Process of preparation of a non-anionic functionalized polymer P3 comprising:

(1) providing a polymer P2 having at least one carbon-carbon double bond, previously prepared by reaction Re1 between a polymer P1 and a compound C1 wherein:

(i) the polymer P1 has at least one functional group chosen from: hydroxy(-OH), thiol (-SH), amine (-NH);
(ii) the compound C1 is selected from the group consisting of compounds of formula I, of formula II, of formula III, and mixtures thereof:

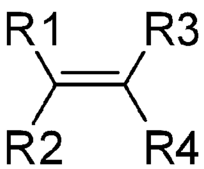 (I) 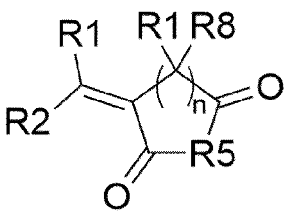 (II) 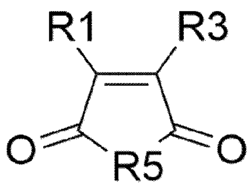 (III),

in which:

- R1, R2 and R3, identical or different, represent a hydrogen atom or a saturated or unsaturated hydrocarbon chain having from 1 to 20 carbon atoms that is linear or branched and may contain one or more heteroatoms selected from the group consisting of oxygen (O), nitrogen (N) or sulfur (S),
- R4 represents a functional group selected from the group consisting of: carboxylic acid (-C(O)OH), a salt of carboxylic acid, anhydride (-C(O)OC(O)R6), acyl halide (-C(O)X in which X is a halogen atom), ester (-C(O)OR7), amide (-C(O)$NH_2$, amide -C(O)N$(R7)_2$, amide -C(O)NHR7), sulfone (-S$(O_2)$OR7), sulfonic acid (-S$(O_2)$OH), a salt of sulfonic acid, phosphonate (-P(O)$(OR7)_2$), phosphonic acid (-P(O)$(OH)_2$), a salt of phosphonic acid, hemi-phosphonate (-P(O)(OH)(OR7)), a salt of hemi-phosphonate,
- R5 represents an atom of oxygen (O), nitrogen (N) or sulfur (S),
- n is an integer between 1 and 3,
- R6 is selected from the group consisting of (a) a saturated or unsaturated, linear or branched, hydrocarbon chain having from 1 to 20 carbon atoms, (b) a 5 or 6-membered aryl, and

(R1)(R2)C=C(R3)-,

wherein R6 may contain one or more heteroatoms selected from the group consisting of oxygen (O), nitrogen (N), sulfur (S) and mixtures thereof,

- R7 represents a saturated or unsaturated, linear or branched hydrocarbon chain having from 1 to 20 carbon atoms or an aryl, optionally substituted or a glycidyl group,

wherein R7 can optionally comprise one or several heteroatoms selected from the group consisting of oxygen (O), sulfur (S), nitrogen (N) and mixtures thereof,

- R8 represents a hydrogen atom or a saturated or unsaturated, linear or branched hydrocarbon chain having from 1 to 20 carbon atoms or an optionally substituted aryl,

wherein R8 can optionally comprise one or more heteroatoms selected from the group consisting of oxygen (O), nitrogen (N) or sulfur (S) and mixtures thereof,

(2) preparing polymer P3 by reaction Re2 between the polymer P2 and a compound C2 wherein the compound C2 is selected from the group consisting of compounds of formula (IV) and salts thereof, compounds of formula (V) and salts thereof, and mixtures thereof:

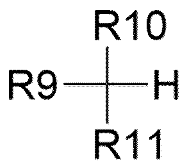 (IV)

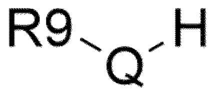 (V)

in which:

- R9, R10 and R11, identical or different, represent, independently from each other, a hydrogen atom; saturated or unsaturated hydrocarbon chain having from 1 to 20 carbon atoms that may contain one or more heteroatoms selected from the group consisting of oxygen (O), nitrogen (N) or sulfur (S) and mixtures thereof, the hydrocarbon groups having from 1 to 20 carbon atoms being optionally substituted with an acid (-C(O)OH) or an ester (-C(O)OR12) function with R12 being a linear or branched hydrocarbon chain having from 1 to 20 carbon atoms or an optionally substituted aryl,

wherein, in formula (IV), at least one of R9, R10 and R11 is an electron-withdrawing group selected from the group consisting of: aldehyde (-CHO), acyl (-COR6), carboxylic acid (-C(O)OH), a salt of carboxylic acid, amide ($-C(O)NH_2$, amide $-C(O)N(R7)_2$, amide - C(O)NHR7), nitrile (-CN), sulfone ($-S(O_2)OR7$), sulfonic acid ($-S(O_2)OH$), a salt of sulfonic acid, phosphonate ($-P(O)(OR7)_2$), phosphonic acid ($-P(O)(OH)_2$), a salt of phosphonic acid, hemi-phosphonate (-P(O)(OH)(OR7)), a salt of hemi-phosphonate, nitro ($-NO_2$), imine (-CH=N-R7);

- Q represents a sulfur (S), an oxygen (O) atom or N-R10.

**2.** Process according to claim 1, ***wherein*** the polymer P1 is a natural polymer.

**3.** Process according to claim 1 or 2, ***wherein*** the polymer P1 is a natural polymer selected from the group consisting of: tamarind gum, guar gum, locust bean gum, tara gum, fenugreek, aloe vera, chia seeds, linseeds, psyllium seeds, quince seeds, xanthan gum, gellan gum, welan gum, rhamsan gum, dextran, curdlan, pullulan, scleroglucan, pectin, schizophyllan, chitin, hydroxyalkylcellulose, arabin, arabinoxylane, curdlan, galactans, pectic galactans, galactomannan, glucomannan, lichenan, mannan, pachyman, rhamnogalacturonan, acacia gum, agar, alginates, carrageenans, chitosan, clavans, hyaluronic acid, heparin, inulin, cellodextrins, carboxy methyl cellulose, cellulose, cellulose derivatives, and mixtures thereof.

**4.** Process according to any one of the preceding claims, ***wherein*** the compound C1 is selected from the group consisting of: methacrylic anhydride, itaconic anhydride, maleic anhydride, acryloyl chloride, acryloyl bromide, methacryloyl chloride, methacryloyl bromide, glycidyl (meth)acrylate, and mixtures thereof.

**5.** Process according to any one of the preceding claims, ***wherein,*** in reaction Re1, a molar ratio between the compound C1 and the at least one functional group of the polymer P1 ranges from 2: 1 to 1:10000.

**6.** Process according to any one of the preceding claims, ***wherein*** the polymer P2 has a degree of substitution by the compound C1 of between 0.0001 and 1.

7. Process according to any one of the preceding claims, ***wherein*** the compound C2 is has a cationic charge.

8. Process according to any one of the preceding claims, ***wherein*** the compound C2 is of formula (V) wherein Q represents a sulfur.

9. Process according to any one of the preceding claims, ***wherein*** the compound C2 comprises at most one thiol group.

10. Process according to any one of the preceding claims, ***wherein*** the compound C2 is selected from the group consisting of:

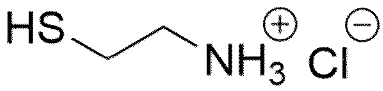
(VII)

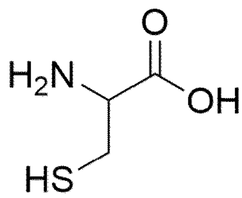
(VIII)

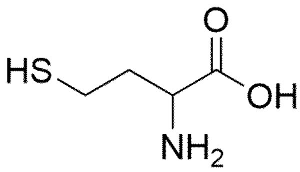
(IX)

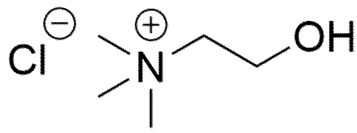
(X)

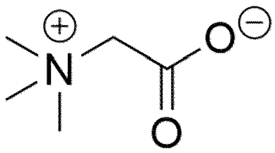
(XI)

11. Process according to any one of the preceding claims, ***wherein,*** in reaction Re2, the polymer P2 and compound C2 have a molar ratio of between 1:2 and 10:1.

12. Process according to any one of the preceding claims, ***wherein*** compound C1 and compound C2 are compounds at least partially of renewable and of non-fossil sources.

13. Process for treating a suspension of solid particles in water or wastewater, comprising contacting said suspension with at least one non-anionic functionalized polymer prepared according to the process of any one of claims 1 to 12.

14. Process for manufacturing a sheet of paper, cardboard or the like, wherein, before a sheet is formed, at least one non-anionic functionalized polymer prepared according to the process of any one of claims 1 to 12 is added to an aqueous suspension of fibers at one or more injection points.

15. Process for conditioning fibers in home care or personal care, comprising contacting said fibers with at least one non-anionic functionalized polymer prepared according to the process of any one of claims 1 to 12.

Europäisches Patentamt
European Patent Office
Office européen des brevets

# EUROPEAN SEARCH REPORT

Application Number
EP 24 30 6756

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | CN 117 815 180 A (BOHUI ZHEJIANG BIOTECHNOLOGY CO LTD)<br>5 April 2024 (2024-04-05)<br>* claim 2 *<br>----- | 1-12<br>13-15 | INV.<br>C08B31/04<br>C08B37/00<br>C08L3/02<br>C08L3/06<br>C08L5/00<br>A61K8/73<br>D21H17/24<br>D21H17/28<br>D21H17/31<br>D21H21/10<br>D21H23/04<br>C02F1/56 |
| A | US 6 063 914 A (WOLF HEIKO [DE] ET AL)<br>16 May 2000 (2000-05-16)<br>* examples 1-8 *<br>----- | 1-15 | |
| A | ROSE JONAS C. ET AL: "Biofunctionalized aligned microgels provide 3D cell guidance to mimic complex tissue matrices",<br>BIOMATERIALS,<br>vol. 163, 1 May 2018 (2018-05-01), pages 128-141, XP093257069,<br>AMSTERDAM, NL<br>ISSN: 0142-9612, DOI: 10.1016/j.biomaterials.2018.02.001<br>* abstract; figure 2A; paragraphs 4.1 and 4.2 *<br>----- | 1-15 | |
| | | | TECHNICAL FIELDS SEARCHED (IPC) |
| | | | C08B<br>C08L<br>A61Q<br>A61K<br>D21H<br>C02F |

~~The present search report has been drawn up for all claims~~

1

| Place of search | Date of completion of the search | Examiner |
|---|---|---|
| Munich | 12 March 2025 | Pellegrini, Paolo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

Europäisches Patentamt
European Patent Office
Office européen des brevets

Application Number
EP 24 30 6756

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-15(partially)

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 24 30 6756

The Search Division considers that the present European patent application does not comply with the requirements of unity of invention and relates to several inventions or groups of inventions, namely:

1. claims: 1-15(partially)

Processes according to claims 1 and 13-15, wherein the polymer P1 is tamarind gum, guar gum, locust bean gum, tara gum, xanthan gum, gellan gum, welan gum, rhamsan gum or acacia gum.

---

2. claims: 1-15(partially)

Processes according to claims 1 and 13-15, wherein the polymer P1 is fenugreek.

---

3. claims: 1-15(partially)

Processes according to claims 1 and 13-15, wherein the polymer P1 is aloe vera.

---

4. claims: 1-15(partially)

Processes according to claims 1 and 13-15, wherein the polymer P1 is chia seeds, linseeds, psyllium seeds or quince seeds.

---

5. claims: 1-15(partially)

Processes according to claims 1 and 13-15, wherein the polymer P1 is dextran.

---

6. claims: 1-15(partially)

Processes according to claims 1 and 13-15, wherein the polymer P1 is curdlan.

---

7. claims: 1-15(partially)

Processes according to claims 1 and 13-15, wherein the polymer P1 is pullulan.

---

8. claims: 1-15(partially)

Processes according to claims 1 and 13-15, wherein the polymer P1 is scleroglucan.

---

Europäisches Patentamt
European Patent Office
Office européen des brevets

# LACK OF UNITY OF INVENTION SHEET B

Application Number
EP 24 30 6756

The Search Division considers that the present European patent application does not comply with the requirements of unity of invention and relates to several inventions or groups of inventions, namely:

9. claims: 1-15(partially)

Processes according to claims 1 and 13-15, wherein the polymer P1 is pectin.

---

10. claims: 1-15(partially)

Processes according to claims 1 and 13-15, wherein the polymer P1 is schizophyllan.

---

11. claims: 1-15(partially)

Processes according to claims 1 and 13-15, wherein the polymer P1 is chitin or chitosan.

---

12. claims: 1-15(partially)

Processes according to claims 1 and 13-15, wherein the polymer P1 is a cellulose or cellulose derivative including hydroxyalkylcellulose and carboxymethylcellulose.

---

13. claims: 1-15(partially)

Processes according to claims 1 and 13-15, wherein the polymer P1 is arabin or arabinoxylane.

---

14. claims: 1-15(partially)

Processes according to claims 1 and 13-15, wherein the polymer P1 is galactan, pectic galactan or galactomannan.

---

15. claims: 1-15(partially)

Processes according to claims 1 and 13-15, wherein the polymer P1 is mannan or glucomannan.

---

16. claims: 1-15(partially)

Processes according to claims 1 and 13-15, wherein the polymer P1 is lichenan.

---

17. claims: 1-15(partially)

Europäisches Patentamt
European Patent Office
Office européen des brevets

# LACK OF UNITY OF INVENTION SHEET B

Application Number
EP 24 30 6756

The Search Division considers that the present European patent application does not comply with the requirements of unity of invention and relates to several inventions or groups of inventions, namely:

Processes according to claims 1 and 13-15, wherein the polymer P1 is pachyman.

---

18. claims: 1-15(partially)

Processes according to claims 1 and 13-15, wherein the polymer P1 is rhamnogalacturonan.

---

19. claims: 1-15(partially)

Processes according to claims 1 and 13-15, wherein the polymer P1 is agar.

---

20. claims: 1-15(partially)

Processes according to claims 1 and 13-15, wherein the polymer P1 is alginate.

---

21. claims: 1-15(partially)

Processes according to claims 1 and 13-15, wherein the polymer P1 is carrageenan.

---

22. claims: 1-15(partially)

Processes according to claims 1 and 13-15, wherein the polymer P1 is clavan.

---

23. claims: 1-15(partially)

Processes according to claims 1 and 13-15, wherein the polymer P1 is hyaluronic acid.

---

24. claims: 1-15(partially)

Processes according to claims 1 and 13-15, wherein the polymer P1 is heparin.

---

25. claims: 1-15(partially)

Processes according to claims 1 and 13-15, wherein the polymer P1 is inulin.

---

Europäisches Patentamt
European Patent Office
Office européen des brevets

# LACK OF UNITY OF INVENTION SHEET B

Application Number
EP 24 30 6756

The Search Division considers that the present European patent application does not comply with the requirements of unity of invention and relates to several inventions or groups of inventions, namely:

26. claims: 1-15(partially)

Processes according to claims 1 and 13-15, wherein the polymer P1 is cellodextrin.

---

## ANNEX TO THE EUROPEAN SEARCH REPORT ON EUROPEAN PATENT APPLICATION NO. EP 24 30 6756

This annex lists the patent family members relating to the patent documents cited in the above-mentioned European search report. The members are as contained in the European Patent Office EDP file on
The European Patent Office is in no way liable for these particulars which are merely given for the purpose of information.

12-03-2025

| Patent document cited in search report | Publication date | Patent family member(s) | Publication date |
|---|---|---|---|
| CN 117815180 A | 05-04-2024 | NONE | |
| US 6063914 A | 16-05-2000 | AT E254633 T1 | 15-12-2003 |
| | | DE 19702641 C1 | 20-08-1998 |
| | | EP 0855405 A1 | 29-07-1998 |
| | | JP H10212302 A | 11-08-1998 |
| | | US 6063914 A | 16-05-2000 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 22254083 A


### Non-patent literature cited in the description


- **M.-P. LABEAU** ; **P. MARION** ; **F. MONNET et al.** Chemicals and Fuels from Bio-Based Building Blocks. Wiley, 2016, 615-642
- Time-resolved Liquid Scintillation Counting. **ROESSLER, N.** ; **VALENTA, R. J.** ; **VAN CAUTER, S.** Liquid Scintillation Counting and Organic Scintillators. Lewis Publishers, 1991, 501-511
- **ALLISON, C. E.** ; **FRANCY, R. J.** ; **MEIJER, H. A. J.** Reference and Intercomparison Materials for Stable Isotopes of Light Elements. *International Atomic Energy Agency, Vienna, Austria, IAEATECHDOC-825*, 1995